# EUROPEAN PATENT APPLICATION

(11) **EP 4 802 985 A1**
(43) Date of publication of application: **09.09.2026**
(21) Application number: 26162037.1
(22) Date of filing: 03.03.2026
(51) Int. Cl.: A61B 1/00, A61B 1/015

(54) **A NOVEL ENDOSCOPIC VALVE**

(30) Priority: 07.03.2025 US 202563768311 P
(71) Applicant: GA HEALTH COMPANY LIMITED, Shatin, N.T. (HK)
(72) Inventor: MCCABE, Ken, Hong Kong (CN); CHAN, Tsan Ho, Hong Kong (CN)
(74) Representative: Ullrich & Naumann PartG mbB

(57) **Abstract**

The present invention is related to an endoscopic valve, adaptable to position in a port of an endoscope for assisting a procedure for performing an endoscopy, comprising: a main body, comprising an internal cavity and a first limiting structure, and being positioned on said port when said valve being positioned in said port; a shaft, partially received in said internal cavity of said main body and profiled into a tubular structure having: at least five recesses, comprising a first recess, a second recess, a third recess, a fourth recess, and a fifth recess sequentially arranged from a top portion to a bottom portion of said shaft on the length side thereof, wherein each of said at least five recesses being fitted with a sealing member; a first fluidic path, defined by at least two openings on the length side of the shaft; a second fluidic path, fluidically connecting with said first fluidic path, defined by an auxiliary opening on said top portion of said shaft fluidically connecting with at least one of said at least two openings, wherein said first fluidic path and at least part of said second fluidic path defining an internal channel inside said tubular structure; and a first limiting structure; a cap removably engaging with said shaft; a resilient member between said cap and said main body, whereby said shaft being adapted to be movable reciprocally between a first position where said first limiting structure of said shaft engaging said first limiting structure of said main body and a second position where said first limiting structure of said shaft disengaging said first limiting structure of said main body; and a shell, adjacent said main body, for fastening said valve to said port; characterized in that, said at least one of said sealing members is configured to comprise a central bore and a flared wing slanting outwardly from the surrounding wall of the central bore of said one of said sealing member.

## Description

### FIELD OF THE INVENTION

The present invention is related to an endoscopic valve, in particular an air water channel valve, a specialized device designed for endoscopic procedures.

### BACKGROUND OF THE INVENTION

During endoscopic examinations, common malfunctions of the air/water valve typically stem from mechanical blockage, seal failure, or improper cleaning and maintenance. The most frequent problem is blockage of the valve body or nozzle, often caused by hardened biological debris such as mucus, blood, or tissue residue after the examination, or by the shedding of residue from chemical disinfectants. This results in insufficient airflow or inability to properly spray water to clean the lens.

Furthermore, worn, deformed, or missing valve sealing elements (such as O-rings) can cause significant leakage, clinically manifested as water dripping or a continuous hissing sound of gas escaping. This not only reduces internal system pressure but can also lead to inaccurate airflow control and even abdominal overinflation. Mechanical "sticking" is also a common obstacle, usually caused by long-term accumulation of residue or lack of lubrication, preventing the valve from quickly returning to its original position after being pressed, resulting in continuous airflow or water supply. If these problems are not addressed through regular functional checks, backflushing, and professional lubrication, they will directly affect the clarity of the physician's observation of the lesion, and may even interrupt the medical procedure due to pressure control failure.

The traditional design of an air water channel valve is characterized by an excessive number of components and a highly complex internal structure, which creates significant challenges during the manufacturing and machining processes. This complexity not only drives up production costs but also results in narrow crevices where surgical debris and biological residues can easily become trapped. These residues pose a severe risk of blockage and, more critically, lead to the survival of bacteria between uses. Because these typical valves are intended for repeated use, the difficulty of thorough sterilization creates a persistent threat of cross-infection between patients. Furthermore, the physical integrity of the device degrades over time due to the stresses of multiple reprocessing cycles. Frequent use leads to progressive mechanical wear and the eventual displacement or failure of critical components, such as rubber O-rings and seals. This structural degradation introduces unpredictable functional risks during endoscopic procedures-most notably unintentional leakage of fluids or gases-which can compromise the surgical field and jeopardize patient safety.

The public needs a simpler design because it directly translates to enhanced patient safety. A design with fewer, more accessible pathways improves reliability, ensuring the device functions correctly during critical procedures and lowers costs for healthcare providers, making care both safer and more affordable. Simplification, in this case, is not a compromise but a significant advancement in medical safety and efficiency.

The present invention at least seeks to address issues of these problems, or at least to provide an alternative to the public.

### SUMMARY OF THE INVENTION

The first aspect of the present invention is related to an endoscopic valve, adaptable to position in a port of an endoscope for assisting a procedure for performing an endoscopy, comprising: a main body, comprising an internal cavity and a first limiting structure, and being positioned on said port when said valve being positioned in said port; a shaft, partially received in said internal cavity of said main body and profiled into a tubular structure having: at least five recesses, comprising a first recess, a second recess, a third recess, a fourth recess, and a fifth recess sequentially arranged from a top portion to a bottom portion of said shaft on the length side thereof, wherein each of said at least five recesses being fitted with a sealing member; a first fluidic path, defined by at least two openings on the length side of the shaft; a second fluidic path, fluidically connecting with said first fluidic path, defined by an auxiliary opening on said top portion of said shaft fluidically connecting with at least one of said at least two openings, wherein said first fluidic path and at least part of said second fluidic path defining an internal channel inside said tubular structure; and a first limiting structure; a cap removably engaging with said shaft; a resilient member between said cap and said main body, whereby said shaft being adapted to be movable reciprocally between a first position where said first limiting structure of said shaft engaging said first limiting structure of said main body and a second position where said first limiting structure of said shaft disengaging said first limiting structure of said main body; and a shell, adjacent said main body, for fastening said valve to said port; characterized in that, one of said sealing members is configured to comprise a central bore and a flared wing slanting outwardly from the surrounding wall of the central bore of said one of said sealing member.

In some embodiments, said flared wing slanting upwards in a direction from said bottom portion to the top portion of said tubular structure.

In some embodiments, said one of said sealing members is the third sealing member counting from said top portion to said bottom portion of said tubular structure.

In some embodiments, the thickness of said flared wing is in a range of 0.2 - 0.5 mm.

In some embodiments, said flared wing is prepared by elastic and/or resilient material selected from a group comprising silicone, rubber, and thermoplastic elastomer (TPE).

In some embodiments, said shaft being manufactured as a single piece.

In some embodiments, said shaft being manufactured in plastic.

In some embodiments, said cap, said main body, and said shell being made of plastic.

In some embodiments, when said valve being positioned in said port, when said shaft being at said first position and when said auxiliary opening being not covered, gas flow in said procedure passing through at least part of said first fluidic path and said second fluidic path.

In some embodiments, when said valve being positioned in said port, when said shaft being at said first position, and when said auxiliary opening being covered, gas flow in said procedure passing through said second fluidic path and through fluid passage between the outer side of the surrounding wall of said shaft and the inner side of the surrounding wall of said port.

In some embodiments, when said valve being positioned in said port, when said shaft being at said second position, and when said auxiliary opening being covered, water flow in said procedure passing through fluid passage between the outer side of the surrounding wall of said shaft and the inner side of the surrounding wall of said port.

In some embodiments, said valve being configured to enable: when said shaft being at said first position and when said auxiliary opening being not covered, gas flow, flowing into said port via a first conduit of said port, leaving said port via a third conduit thereof through said at least part of said first fluidic path and said second fluidic path.

In some embodiments, said valve being configured to enable: when said shaft being at said first position and when said auxiliary opening being covered, gas flow, flowing into said port via a first conduit of said port, leaving said port via a third conduit through said first fluidic path and fluid passage between the outer side of the surrounding wall of said shaft and the inner side of the surrounding wall of said port.

In some embodiments, said valve being configured to enable: when said shaft being at said second position and when said auxiliary opening being covered, water flow, flowing into said port via a second conduit of said port, leaving said port via a fourth conduit through fluid passage between the outer side of the surrounding wall of said shaft and the inner side of the surrounding wall of said port.

In some embodiments, wherein said valve is an air water channel valve.

The second aspect of the present invention is related to a method to provide an endoscopic valve of the first aspect of the present invention.

### DESCRIPTION OF THE DRAWINGS

Some embodiments of the present invention will now be explained, with reference to the accompanied drawings, in which:-
- Figure 1: is a perspective view of a first embodiment of an endoscopic valve of the present invention;
- Figure 2A: is a first sectional view of the first embodiment of the endoscopic valve of the present invention;
- Figure 2B: is a second sectional view of the first embodiment of the endoscopic valve of the present invention;
- Figure 3A: is a sectional view of the first embodiment of the endoscopic valve mounted into a port of an endoscope, in which a cap of the endoscopic valve is unpressed and a first opening of the cap is uncovered;
- Figure 3B: is a sectional view of the first embodiment of the endoscopic valve mounted into the port of the endoscope, in which the cap of the endoscopic valve is unpressed but the first opening of the cap is covered by a finger of a user;
- Figure 3C: is a sectional view of the first embodiment of the endoscopic valve mounted into the port of the endoscope, in which the cap of the endoscopic valve is pressed and the first opening of the cap remains covered by the finger of the user;
- Figure 4A: is a sectional view of an endoscopic valve in prior art mounted
- Figure 4B: into a port of an endoscope, in which a cap of the endoscopic valve is unpressed and a first opening of the cap is uncovered; is a sectional view of the endoscopic valve in figure 4A mounted into the port of the endoscope, in which the cap of the endoscopic valve is unpressed but the first opening of the cap is covered by a finger of a user; and
- Figure 4C: is a sectional view of the endoscopic valve in figure 4A mounted into the port of the endoscope, in which the cap of the endoscopic valve is pressed and the first opening of the cap remains covered by the finger of the user.

### DETAILED DESCRIPTION OF PREFERRED EMBODIMENTS OF THE INVENTION

The present invention is now presented by way of examples with reference to the figures in the following paragraphs. Objects, features, and aspects of the present disclosure are disclosed in or are apparent from the following description. It should be understood by one of ordinary skilled in the art that the following description is a description of exemplary embodiments only, and is not intended as limiting the broader aspects of the present disclosure, which broader aspects are embodied in the exemplary constructions.

It should be noted that, unless otherwise defined, the technical terms or scientific terms used in the embodiments of the present invention shall have the usual meanings understood by person with ordinary skills in the art to which the present invention belongs. "First", "second" and similar expression used in the embodiments of the present invention do not indicate any order, quantity or importance, but are only used to distinguish different components. "Front", "rear", "left", "right", "upper", and "lower" and other terms indicating orientation or similar terms are only described for the exemplary relative positional relationship shown in the drawings to facilitate the understanding. It does not limit the disclosed components in the present invention can only follow this specific relative positional relationship.

For brevity's sake, the operation of an endoscope, the connection means of the endoscope to a water source, air source, and suction source are not described and explained in this specification, as this information constitute the state of art.

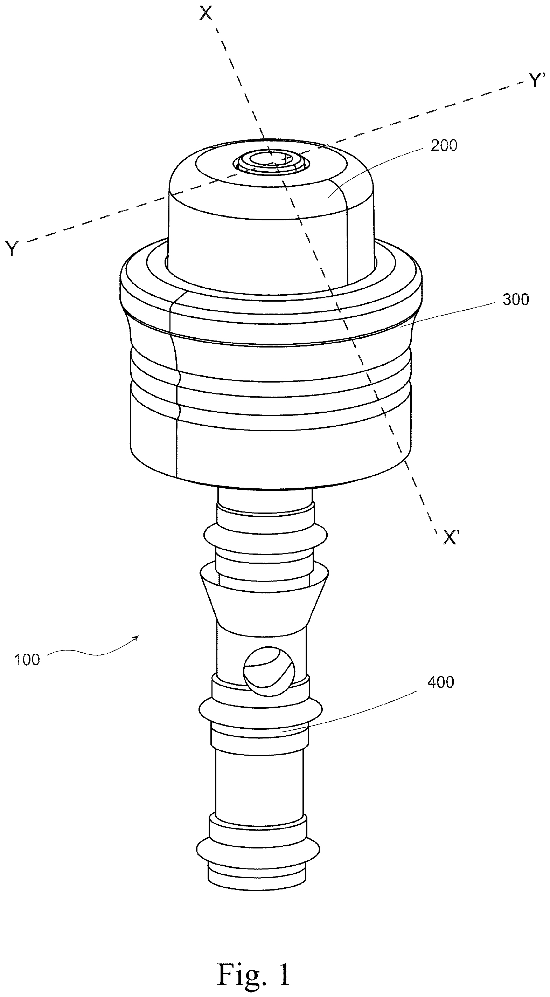

Figure 1 is a perspective view of a first embodiment of an endoscopic valve of the present invention, which is an air water channel valve 100 that comprises a cap 200, a shell 300, and a shaft 400.

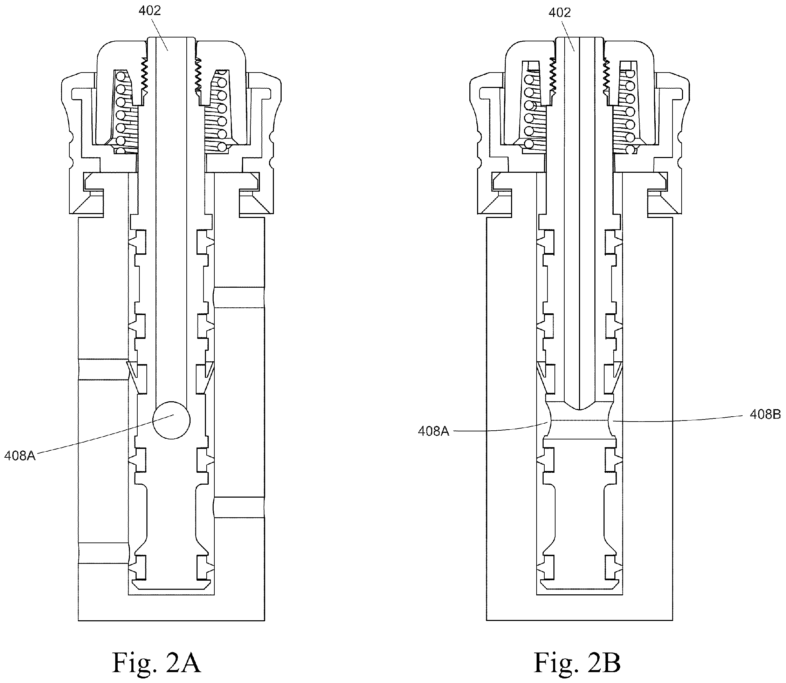

Figure 2A is a first sectional view, along dotted line YY' in figure 1, of the first embodiment of the endoscopic valve of the present invention.

Figure 2B is a second sectional view, along dotted line XX' in figure 1, of the first embodiment of the endoscopic valve of the present invention.

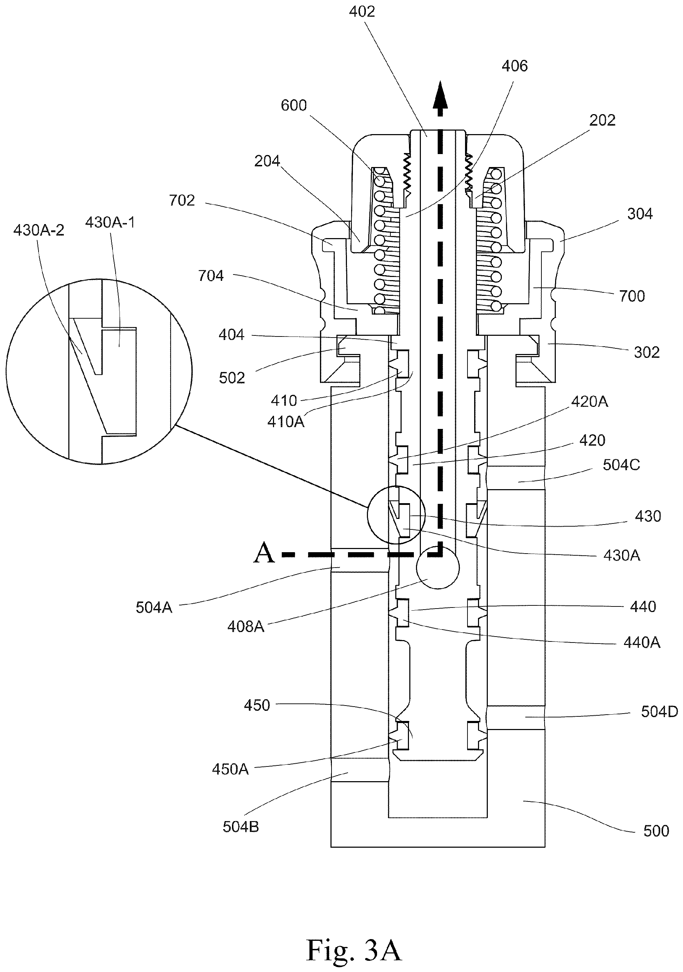

Figures 3A-3C are sectional views of the first embodiment of the air water channel valve 100 that is removably mounted into a port 500 which belongs to part an endoscope.

Figure 3A illustrates that the air water channel valve 100 further comprises a main body 700 being designed to comprise an open top and an open bottom, which defines an internal cavity within the main body 700 that allows a portion of the shaft 400 to pass through or houses a portion of the shaft 400. The main body 700 is designed to provide a first limiting structure 702 preferably having a right-angled shape and extending, preferably outwardly, from the surrounding wall of the main body 700, and a second limiting structure 704 preferably having a right-angled shape and extending, preferably inwardly, from the surrounding wall of the main body 700. It shall be important to note that the shape and/or orientation of the first and second limiting structures 702 and 704 disclosed in figures is an example only; those skilled in the art could configure it according to actual needs.

As shown in figure 3A, the cap 200 is provided with a first limiting structure 202 and a second limiting structure 204, and a central aperture on a top plate of the cap 200.

As illustrated in the accompanying figures, the rim of the central aperture is configured to provide elongated leg defining said first limiting structure 202 that extend downward from the top plate of the cap 200. The leg is configured such that it defines a cavity or channel of the cap 200 to receive a portion of the upper section of the shaft 400. More specifically, when the air water channel valve 100 is inserted into the port 500, the leg extends from the underside of the cap's top surface in the direction of the port 500.

Advantageously, the inner surface of the elongated leg is equipped with engagement members such as threaded screws or threads. These threads are designed to engage with corresponding threads (examples of engagement members) provided on the outer peripheral tip or upper end of the shaft 400. This threaded engagement between the cap 200 and the shaft 400 allows the cap 200 to be securely fastened onto the shaft 400, ensuring a stable connection during use. However, it is important to note that the use of threaded screws is merely an example of a possible coupling mechanism. The cap 200 may also be fastened, preferably removably fastened, to the upper portion of the shaft 400 through alternative means, such as a snap-fit connection or a tight-fit engagement, depending on the design requirements or the intended application.

A resilient member 600, in a form of coil spring, is positioned between the cap 200 and the main body 700, in particular between the cap 200 and the main body's second limiting structure 704. This is important to note that coil spring is an example of the resilient member 600 only and shall not be construed as any limitation to the resilient member 600.

With the aid of the resilient member 600, the cap 200 can move reciprocally inside the internal cavity of the main body 700 with respect to the main body 700 in a first position where the cap 200 is further from the second limiting structure 704 and a second position where the cap 200 is closer to the second limiting structure 704. To be specific, when the cap 200 is advanced closer to the second limiting structure 704, the advancement is limited by the interaction between the second limiting structure 204 and the second limiting structure 704; when the cap 200 is retracted away from the second limiting structure 704, the retraction is limited by the interaction between a first limiting structure 404 of the shaft 400 (will be explained later) and the second limiting structure 704 of the main body 700.

The shaft 400 has an elongate tubular structure and comprises an upper portion and a lower portion.

The shaft 400 is provided with the first limiting structure 404 outwardly protruding from the peripheral of the shaft 400. Preferably, the first limiting structure 404 is configured as a right-angled profile that is complementary to the shape of the second limiting structure 704 of the main body 700. It shall be important to note that the shape and/or orientation of the first limiting structure 404 disclosed in figures is an example only; those skilled in the art could configure it according to actual needs. As long as the interaction of the second limiting structure 704 and the first limiting structure 404 limits the movement of the shaft 400, it belongs to the gist of the limiting structure 704 and the first limiting structure 404.

The shaft 400 is also provided with a second limiting structure 406 outwardly protruding from the peripheral of the upper portion of the shaft 400. Preferably, the second limiting structure 406 is configured as a right-angled profile that is complementary to the shape of a first limiting structure 202 of the cap 200. It shall be important to note that the shape and/or orientation of the second limiting structure 406 and the first limiting structure 202 disclosed in figures is an example only; those skilled in the art could configure it according to actual needs. As long as the second limiting structure 406 and the first limiting structure 202 are complementary, it belongs to the gist of the limiting structure 406 and the first limiting structure 202.

The interaction between the second limiting structure 704 of the main body 700 and the first limiting structure 404 of the shaft 400 defines the upper portion and the lower portion of the shaft 400, in which the portion of the shaft 400 above said interaction is the upper portion of the shaft 400 while the portion of the shaft 400 below said interaction is the lower portion of the shaft 400. Referring to figure 3A, the upper portion of the shaft 400 is partially housed inside the internal cavity of the main body 700. To be more specific, the upper portion of the shaft 400 is partially housed inside the internal cavity of the main body 700 and the internal cavity of the cap 200, and one end of the upper portion of the shaft 400 is arranged flush with the top plate of the cap 200 or beyond the top plate of the cap 200 or below the top plate of the cap 200. Although the accompanying figures show that the one end of the upper portion of the shaft 400 is arranged beyond the top plate of the cap 200, it shall not be construed as any limitation to the present invention.

The port 500 has a receptacle-like structure with an open-top internal cavity defined by the surrounding wall and bottom of the port 500. The port 500 is configured to receive the air water channel valve 100 and is provided with a limiting structure 502. Preferably, the limiting structure 502 is configured as a right-angled profile that is complementary to the shape of the second limiting structure 704 of the main body 700 and/or the first limiting structure 404 of the shaft 400. It shall be important to note that the shape and/or orientation of the limiting structure 502 disclosed in figures is an example only; those skilled in the art could configure it according to actual needs. As long as the interaction of the limiting structure 502 and the second limiting structure 704 of the main body 700 aids in the assembling between the main body 700 and the port 500 and/or as long as the interaction of the limiting structure 502 and the first limiting structure 404 of the shaft 400 aids in the assembling between the shaft 400 and the port 500, it belongs to the gist of these limiting structures.

The main body 700 and port 500 are assembled by disposing the limiting structure 704 of the main body 700 on the limiting structure 502 of the port 500. However, the assembling between the main body 600 and the port 500 can be realized by any other means, for example, via a snug fit.

The shell 300 is profiled to provide a first limiting structure 302 inwardly protruding from the peripheral of the surrounding wall of the shell 300. The shape of the first limiting structure 302 is complementary to the shape of the limiting structure 502 of the port 500, which aids to assist the assembling of the shell 300 to the port 500. It shall be important to note that the shape and/or orientation of the first limiting structure 302 disclosed in figures is an example only; those skilled in the art could configure it according to actual needs. As long as the interaction of the first limiting structure 302 and the limiting structure 502 facilitates the assembling of the shell 300 to the port 500, it belongs to the gist of the limiting structure.

The shell 300 is also profiled to provide a second limiting structure 304 inwardly protruding from the peripheral of the surrounding wall of the shell 300. The shape of the second limiting structure 304 is complementary to the shape of the first limiting structure 702 of the main body 700, which aids to assist the assembling of the shell 300 to the main body 700 and/or assembling of the main body 700 to the port 500. It shall be important to note that the shape and/or orientation of the second limiting structure 304 disclosed in figures is an example only; those skilled in the art could configure it according to actual needs. As long as the interaction of the second limiting structure 304 and the first limiting structure 702 facilitates the assembling of the shell 300 to the main body 700 and/or the assembling of the main body 700 to the port 500, it belongs to the gist of the limiting structure.

Advantageously, the inner wall of the shell 300 and the outer wall of the main body 700 are profiled with complementary contours and/or patterns for aiding the assembly between the main body 700 and the shell 300. For example, as illustrated in figure 3A, the shell 300 is internally provided with the limiting structures 302 and 304 having shape dimensioned to be complementary to the shape of the limiting structure 502 of the port 500 and the first limiting structure 702 of the main body 700, respectively. It shall be important to note that the shapes of these limiting structures disclosed in figures are an example only; those skilled in the art could configure it according to actual needs. As long as the interaction of these limiting structures aids in the assembling between the main body 700 and the shell 300 and/or the port 500 and the shell 300, it belongs to the gist of the limiting structure.

The shaft 400 is at least partially housed inside an accommodation cavity defined by the internal cavity of the main body 700 and the internal cavity of the port 500. The depth of the internal cavity of the port 500 is deeper than the length of the lower portion of the shaft 400, such that the lower portion of the shaft 400 is movable in the internal cavity of the port 500between the second limiting structure 704 of the main body 700 and the bottom of the port 500. To be more specific, with the aid of the resilient member 700, disposed between the cap 200 and the main body 700, the lower portion of the shaft 400 is reciprocally movable between the second limiting structure 704 of the main body 700 and the bottom of the port 500. Advantageously, regardless whether the cap 200 is pressed or not pressed, the bottom of the shaft 400 does not engage with the bottom of the port 500.

The position of the cap 200 with respect to the port 500 controls a pressing action. When a user presses the cap 200, it actuates the lower portion of the shaft 400 to move from a first position where the second limiting structure 704 of the main body 700 interacts with the first limiting structure 404 of the shaft 400 to a second position where the second limiting structure 704 of the main body 700 does not interact with the first limiting structure 404 of the shaft 400. By default, when the cap 200 is unpressed, the cap 200 is at its first position; when the cap 200 is pressed, the cap 200 is at its second position. The position of the cap 200 with respect to the port 500 also defines the configuration of the air water channel valve 100, in which the first position of the cap 200 defines the first configuration of the air water channel valve 100 and the second position of the cap 200 defines the second configuration of the air water channel valve 100.

The shaft 400 features a sophisticated sealing system comprising five precisely machined recesses-designated as a first recess 410, a second recess 420, a third recess 430, a fourth recess 440, and a fifth recess 450-arranged sequentially along the vertical axis of the shaft 400, in which the first recess 410 is at the top among these recesses. Preferably, the first recess 410 is always in the internal cavity defined by the port 500 during the operation of an endoscope. It shall be important to note that the number of recesses provided to the shaft 400 is at least five. Each recess is annularly, preferably continuous, around the tubular structure of the shaft 400 and hosts a plurality of sealing members: a first sealing member 410A, a second sealing member 420A, a third sealing member 430A, a fourth sealing member 440A, and a fifth sealing member 450A, respectively.

Advantageously, the first sealing member 410 is configured to comprise a central bore and an annular protrusion projecting outwardly from the surrounding wall of the central bore of the first sealing member 410. The annular protrusion is sized and dimensioned fit to act against the inner side of the surrounding wall of the port 500. It shall be important to note that the annular protrusion is sufficiently long to act against the inner side of the surrounding wall of the port, avoiding contact between the surface of the tubular structure of the shaft and the surface of the inner side of the surrounding wall of the port thus preventing wearing off the surface of the inner side of the surrounding wall of the port. Depending on the application and design, any one of these sealing members 420A, 430A, 440A, and 450A can be configured as any form as long as it can be functioned as a sealing member. As long as the sealing member is sized and dimensioned to act against the inner side of the surrounding wall of the port, avoiding contact between the surface of the tubular structure of the shaft and the surface of the inner side of the surrounding wall of the port thus preventing wearing off the surface of the inner side of the surrounding wall of the port, such alternative of the sealing member also falls within the gist of the invention.

Any of the second sealing member 420, the fourth sealing member 440, and the fifth sealing member 450 can be advantageously configured the same as the first sealing member 410.

Advantageously, the third sealing member 430 is configured to comprise a central bore 430A-1 and a flared wing 430A-2 slanting outwardly from the surrounding wall of the central bore of the third sealing member 430, such that a first end of the flared wing 430A-2 remains connecting with the surrounding wall of the central bore 430A-1 while a second end of the flared wing 430A-2, opposite the first end of the flared wing 430A-2, acts against the inner side . As shown in figure 3A, the slanting is oriented upwards in a direction from the fourth recess to the second recess, i.e., the first end of the flared wing 430A-2 is positioned closer to the fourth recess while the second end of the flared wing 430A-2 is positioned closer to the second recess. The flared wing is sized and dimensioned fit to act against the inner side of the surrounding wall of the port 500. More advantageously, the flared wing is thin, preferably in a range of 0.2 - 0.5 mm. Most advantageously, the third sealing member is prepared by elastic and/or resilient material selected from a group comprising silicone, rubber, and thermoplastic elastomer (TPE). The configuration of the flared wing allows it to be deflected under air flowing, of which the operation is explained below.

When the air water channel valve 100 is mounted to the port 500, the outer side of the surrounding wall of the shaft 400 and the inner side of the surrounding wall of the port 500 leaves a narrow channel running their entire lengths, which functions as a dedicated fluid passage. In this specification, "narrow channel between the outer side of the surrounding wall of the shaft 400 and the inner side of the surrounding wall of the port 500" and "dedicated fluid passage" are interchangeable. The sealing members 410A, 420A, 430A, 440A, and 450A, made of resilient materials and mounted to their respective recesses of the tubular structure of the shaft 400, acts against the inner side of the surrounding wall of the port 500 thus blocking and/or impeding the narrow channel, i.e., the dedicated fluid passage. It shall be important to understand that "the sealing member acts the inner side of the surrounding wall of the port" in context can also be understood as "the inner side of the surrounding wall of the port biases or presses or compresses the sealing member". When the sealing member is biased, it provides a sealing effect.

Integral to the shaft's function is an internal channel inside the tubular structure of the shaft 400, which serves as a multifunctional fluid conduit. It functions as a controlled passage for fluids, likely facilitating the directed flow of fluids during operation of an endoscope. The essence of the internal channel is to provide a tunnel with at least a first opening 402 and a second opening 408A, and a third opening 408B, allowing fluidic flow. To be more specific, controlling the air flow and water flow during the endoscopy procedures. The second opening 408A and the third opening 408B defines a first fluidic path. The first opening 402 together with the second opening 408A and/or the third opening 408B defines a second fluidic path which fluidically connects with the first fluidic path. It shall be understood that the first fluidic path and at least part of the second fluidic path together define part of the internal channel inside said tubular structure of the shaft. Advantageously, the first fluidic path and the second fluidic path together define the internal channel inside said tubular structure of the shaft. Preferably, the second fluidic path traverses the zones associated with a point - between the third recess 430 and the fourth recess 440 - and a point - the first opening 402 adjacent or at the top plate of the shaft 400. Advantageously, the second opening 408A, and the third opening 408B are positioned higher than the fourth recess 440 but lower than the third recess 430. However, it shall be important to note that these positions of the second opening 408A and the third opening 408B shall not be construed as limitations to the present invention. It shall be important to note that although the internal channel is an inverted T-shaped in figures, it is for illustration only. As long as an internal channel that has at least two openings, which are provided on the length side of the tubular structure of the shaft, and one opening adjacent or at the top plate of the shaft, the internal channel also falls into the gist of the internal channel. In some embodiments, the shape of the internal channel can be Y-shaped, inverted A-shaped, or any shape. In some embodiments, the internal channel has three, four, five or even more openings according to actual needs. In some embodiments, the position of the second and third openings 408A and 408B can be configured at different height on the length side of the shaft.

The surrounding wall of the port 500 is profiled to provide a first conduit 504A for receiving air flow from an air source for the endoscope, a second conduit 504B for receiving water flow from a water source for the endoscope, a third conduit 504C which is an outlet for air, and a fourth conduit 504D which is an outlet for water. It shall be important to understand that the use of air and water is for explanation the operation of the air water channel valve and shall not be construed as limitation to the use of the air water channel valve. In some embodiments, the first conduit 504A can be configured for receiving water flow from a water source for the endoscope, the second conduit 504B can be configured for receiving air flow from an air source for the endoscope. In some embodiments, the third conduit 504C is an outlet for water, and the fourth conduit 504D is an outlet for air. Depending on actual use, the third conduit 504C and the fourth conduit 504D can each be configured as an outlet for both air and water. This means that the third conduit 504C and the fourth conduit 504D are not limited to a single type of fluid. It shall also be important to understand that "conduit" in context means a channel or tunnel or through-opening allowing fluid, i.e., liquid and/or air, passing through. "Fluid" in this specification may refer to liquid and/or air. For example, the first conduit 504A is for allowing fluid to pass through the surrounding wall of the port 500. It is important to note that the conduit shall not be constrained as any shape.

When the air water channel valve 100 is mounted to the port 500, the operation of the air water channel valve 100, i.e., pressing or not pressing the cap 200 of the air water channel valve 100, operably allows the first conduit 504A and the second conduit 504B to allow air and water, respectively, to pass through the air water channel valve 100 to the third conduit 504C and the fourth conduit 504D, respectively.

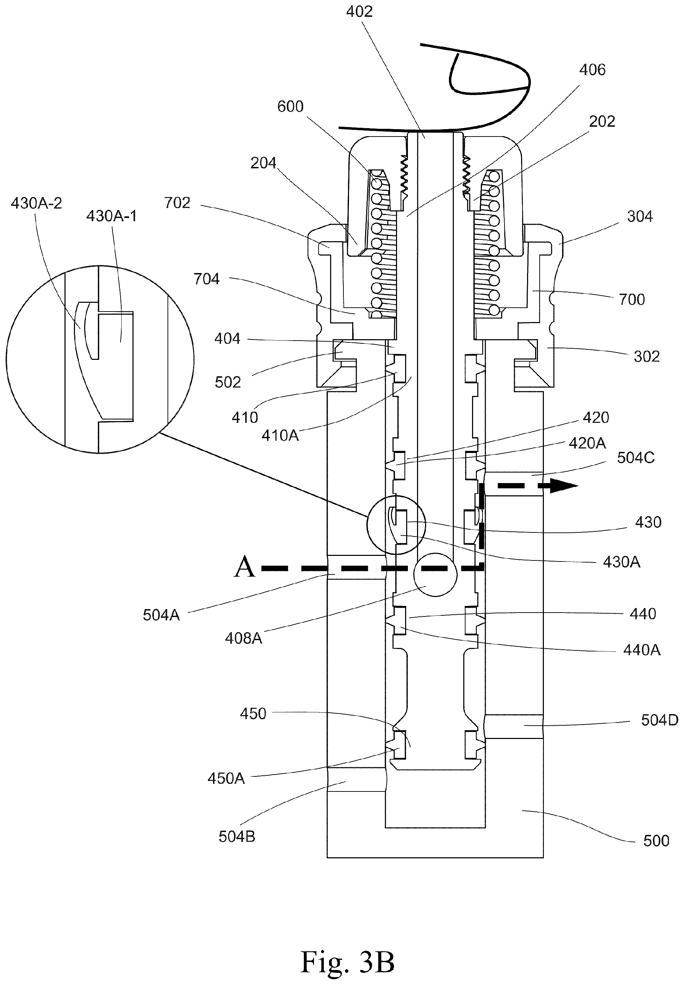

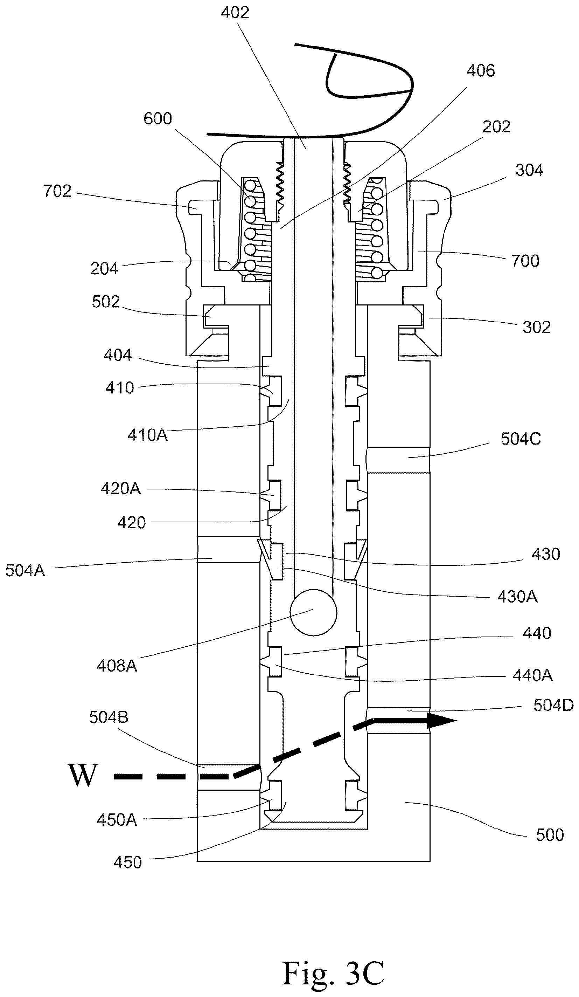

Figures 3A-3C depicts different operations on the endoscope mounted with the air water channel valve 100, in which figure 3A depicts the cap 200 is unpressed, in particular the first opening 402 of the cap 200 is uncovered; figure 3B depicts the first opening 402 is covered by a finger of a user but the cap 200 is unpressed; and figure 3C depicts the first opening 402 is covered by a finger of a user but the cap 200 is pressed.

In figure 3A, since the first opening 402 is not covered and the first sealing member 410A, the second sealing member 420A, the third sealing member 430A, the fourth sealing member 440A, and the fifth sealing member 450A act against the inner side of the surrounding wall of the port 500, thus these five sealing members block and/or impede their respective narrow channels, i.e., the dedicated fluid passages. Accordingly, airflow A is only permitted to flow from the first conduit 504A to the first opening 402 of the shaft 400, via at least part of the first fluidic path and the second fluidic path.

Figure 3B illustrates that a user's finger is placed over the first opening 402. In figure 3B, the first sealing member 410A, the second sealing member 420A, the third sealing member 430A, the fourth sealing member 440A, and the fifth sealing member 450A remains acting against the inner side of the surrounding wall of the port 500, thus these five sealing members remains blocking and/or impeding their respective narrow channels, i.e., the dedicated fluid passages. Technically, the flushed-in air bifurcates into two paths: the first path - the flushed air flows from the first conduit 504A to the dedicated fluid passage between the outer side of the surrounding wall of the shaft 400 and the inner side of the surrounding wall of the port 500, then to the first fluidic path defined by the second opening 408A, then to the dedicated fluid passage between the outer side of the surrounding wall of the shaft 400 and the inner side of the surrounding wall of the port 500, then leaves the port 500 via the third conduit 504C; and the second path - the flushed air flows from the first conduit 504A to the first opening 402 of the shaft 400, as described in figure 3A, which provides no substantial function and effect in the step of air flushing. Technically, airflow fails to pass through the second fluidic path as the first opening 402 is blocked. Therefore, airflow is only permitted to flow from the first conduit 504A to the third conduit 504C. Since almost the entire portion of airflow opts to pass through the first path, the pressure of the airflow is high enough to deflect the flared wing of the third sealing member towards the surrounding wall of the central bore of the third sealing member 430, thus airflow is only permitted to flow from the first conduit 504A to the third conduit 504C. Contrary to the illustration in figure 3A in which almost the entire portion of airflow flows from the first conduit 504A to the first opening 402 of the shaft 400, there is still a trace amount of airflow attempts to pass through the third sealing member 430A. However, since the amount of airflow attempts to pass through the third sealing member 430A is trace, its air pressure is not strong enough to deflect the flared wing of the third sealing member towards the surrounding wall of the central bore of the third sealing member 430.

In figures 3A and 3B, since the five sealing members remains blocking and/or impeding their respective narrow channels, i.e., the dedicated fluid passages, it can prevent air from leaving the internal cavity of the port 500 through the dedicated fluid passages and air can only be directed to flow through the air water channel valve 100 with path as described above.

Figure 3C illustrates that the user's finger presses the cap 200 downwards, i.e., from its first position to its second position. Water W flushes into the second conduit 504B then leaves therefrom, then enters the narrow channel between the exit of the second conduit 504B and cylindrical profile of the shaft 400. Advantageously, the cylindrical profile of the shaft 400 at a position between the fourth sealing member 440A and the fifth sealing member 450A is reduced, thus allowing fluid such as water leaving from the second conduit 504B enters the narrow channel between the exit of the second conduit 504B and cylindrical profile of the shaft 400. Eventually, water leaves the port 500 via the fourth conduit 504D. Since the first sealing member 410A, the second sealing member 420A, the third sealing member 430A, the fourth sealing member 440A, and the fifth sealing member 450A remains acting against the inner side of the surrounding wall of the port 500, thus these five sealing members remains blocking and/or impeding their respective narrow channels, i.e., the dedicated fluid passages. As a result, water is not permitted to flow the narrow channel and/or the dedicated fluid passage.

Subject to the operation procedures, airflow from the first conduit 504A to the third conduit 504C and/or to the first opening 402 through the internal channel is/are permitted; and/or water flow from the second conduit 504B to the fourth conduit 504D is/are permitted.

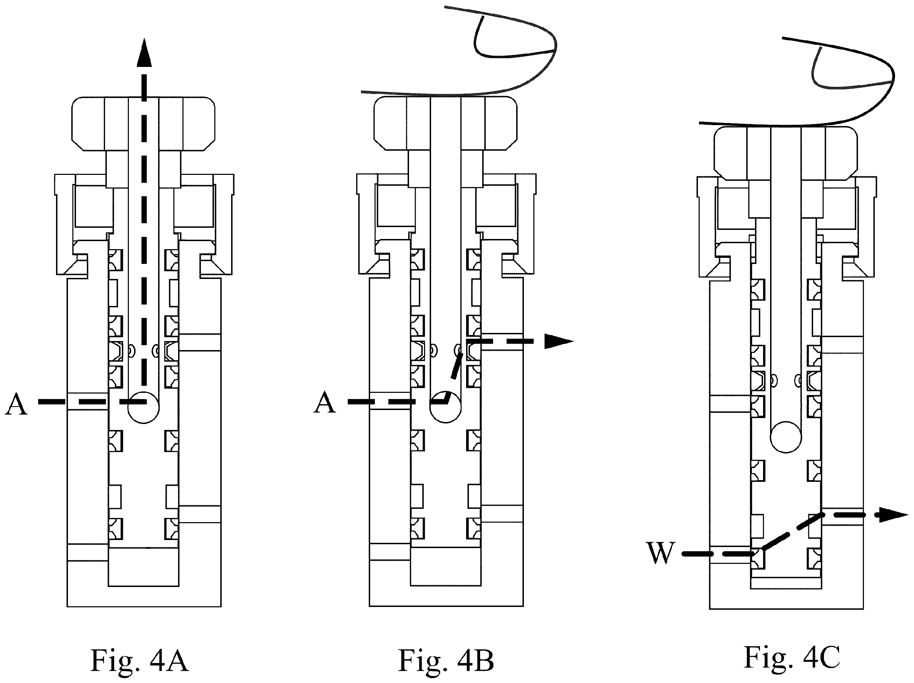

The present invention introduces a significant advancement over conventional shaft designs commonly used in the prior art, such as those illustrated in figure 4A through figure 4C. Traditionally, these shafts are composed of multiple assembled components, typically fabricated from metal. While metal offers strength and durability, it also introduces several drawbacks in terms of manufacturing and operational efficiency. The multi-piece construction inherently increases the complexity of the production process, requiring precise machining, alignment, and assembly of the various components, each of which contributes to a higher overall production cost. Furthermore, the necessity for assembling multiple parts introduces additional steps, potentially increasing the chance of manufacturing defects and prolonging production timelines. One of the specific features of the prior art shaft design involves the presence of six recesses distributed along its length. Each of these recesses is designed to accommodate a sealing member, which is crucial for maintaining the integrity of gas or fluid control within the device (endoscope). However, this configuration not only requires the creation of six precision-formed recesses, but it also demands the careful installation of six individual sealing members-each of which must meet stringent tolerances to ensure proper functionality. Additionally, the tubular structure of the shaft in the prior art includes a specialized through-channel located adjacent to the third recess from the top. This channel permits gas flow through the shaft but represents another complex feature that must be accurately machined, further complicating the production process and introducing another point of potential failure. In contrast, the design described in the present invention achieves a notable simplification. The number of recesses required on the shaft is reduced from six to five, thereby eliminating one sealing member and one associated recess. More importantly, the design omits the additional through-channel entirely, removing the need for a precision-formed gas passage within the shaft. This reduction in structural complexity directly translates to a more streamlined manufacturing workflow, lowering both material and labor costs while improving overall production efficiency. Beyond structural simplification, the present invention introduces a fundamental shift in material choice. Unlike the metal shafts of the prior art, the new design allows for the use of plastic materials. This change is particularly advantageous in clinical and laboratory environments, where hygiene and the risk of cross-contamination are paramount concerns. By enabling the shaft to be manufactured from plastic, the device can be designed for single-use applications. This disposable nature eliminates the need for the rigorous cleaning and sterilization procedures required for reusable metal components, which are both time-consuming and resource-intensive. Moreover, this innovation extends beyond the shaft itself. In some embodiments, the cap and the main body of the device can also be fabricated from plastic, allowing the entire assembly to be disposable. This not only simplifies inventory and logistics but also enhances safety by ensuring that each use involves a sterile, uncontaminated device. From a clinical standpoint, this shift reduces the reliance on sterilization protocols and minimizes the risk of infection or cross-contamination between patients. In summary, the present invention represents a substantial improvement over existing shaft designs by simplifying manufacturing, reducing cost, and enhancing safety and hygiene. The reduction in component complexity, the elimination of a gas through-channel, and the adoption of plastic materials for single-use applications collectively contribute to a more efficient, cost-effective, and clinically advantageous solution.

The present invention overcomes the aforementioned limitations by introducing a streamlined structural design that significantly reduces the total number of components and simplifies the internal architecture. By eliminating the intricate crevices characteristic of traditional valves, this optimized geometry facilitates more effective cleaning and ensures comprehensive sterilization, thereby neutralizing the risks of biological residue accumulation and patient cross-infection. Furthermore, the simplified mechanical framework enhances the device's structural durability, allowing it to withstand the rigors of repeated reprocessing without the progressive wear or seal displacement common in conventional models. This increased reliability ensures consistent, leak-free performance during endoscopic procedures, providing a safer and more cost-effective solution that maintains functional integrity over an extended operational lifespan.

The above-described shall not be interpreted to be restricted by the examples or figures only. It is to be expressly understood, however, that such modifications and adaptations are within the scope of invention in this aspect. For instance, features illustrated or described as part of one embodiment can be used on another embodiment to yield a still further embodiment. Thus, it is intended that the present disclosure cover such modifications and variations and their equivalents.

## Claims

1. An endoscopic valve, adaptable to position in a port of an endoscope for assisting a procedure for performing an endoscopy, comprising:
a main body, comprising an internal cavity and a first limiting structure, and being positioned on said port when said valve being positioned in said port;
a shaft, partially received in said internal cavity of said main body and profiled into a tubular structure having:
- at least five recesses, comprising a first recess, a second recess, a third recess, a fourth recess, and a fifth recess sequentially arranged from a top portion to a bottom portion of said shaft on the length side thereof, wherein each of said at least five recesses being fitted with a sealing member;
- a first fluidic path, defined by at least two openings on the length side of the shaft;
- a second fluidic path, fluidically connecting with said first fluidic path, defined by an auxiliary opening on said top portion of said shaft fluidically connecting with at least one of said at least two openings, wherein said first fluidic path and at least part of said second fluidic path defining an internal channel inside said tubular structure; and
- a first limiting structure;
a cap removably engaging with said shaft;
a resilient member between said cap and said main body, whereby said shaft being adapted to be movable reciprocally between a first position where said first limiting structure of said shaft engaging said first limiting structure of said main body and a second position where said first limiting structure of said shaft disengaging said first limiting structure of said main body; and
a shell, adjacent said main body, for fastening said valve to said port;
**characterized in that**,
at least one of said sealing members is configured to comprise a central bore and a flared wing slanting outwardly from the surrounding wall of the central bore of said one of said sealing member.

2. The valve according to claim 1, wherein said flared wing slanting upwards in a direction from said bottom portion to the top portion of said tubular structure.

3. The valve according to claim 1, wherein said at least one of said sealing members is the third sealing member counting from said top portion to said bottom portion of said tubular structure.

4. The valve according to claim 1, wherein the thickness of said flared wing is in a range of 0.2 - 0.5 mm.

5. The valve according to claim 1, wherein said flared wing is prepared by elastic and/or resilient material selected from a group comprising silicone, rubber, and thermoplastic elastomer.

6. The valve according to claim 1, wherein said shaft being manufactured as a single piece.

7. The valve according to claim 1, wherein said shaft being manufactured in plastic.

8. The valve according to claim 1, wherein said cap, said main body, and said shell being made of plastic.

9. The valve according to claim 1, when said valve being positioned in said port, when said shaft being at said first position, and when said auxiliary opening being not covered:
gas flow in said procedure passing through at least part of said first fluidic path and said second fluidic path.

10. The valve according to claim 9, when said valve being positioned in said port, when said shaft being at said first position, and when said auxiliary opening being covered:
gas flow in said procedure passing through said second fluidic path and through fluid passage between the outer side of the surrounding wall of said shaft and the inner side of the surrounding wall of said port.

11. The valve according to claim 10, when said valve being positioned in said port, when said shaft being at said second position, and when said auxiliary opening being covered:
water flow in said procedure passing through fluid passage between the outer side of the surrounding wall of said shaft and the inner side of the surrounding wall of said port.

12. The valve according to claim 9, said valve being configured to enable, when said shaft being at said first position and when said auxiliary opening being not covered:
gas flow, flowing into said port via a first conduit of said port, leaving said port via a third conduit thereof through said at least part of said first fluidic path and said second fluidic path.

13. The valve according to claim 10, said valve being configured to enable, when said shaft being at said first position and when said auxiliary opening being covered:
gas flow, flowing into said port via a first conduit of said port, leaving said port via a third conduit through said first fluidic path and fluid passage between the outer side of the surrounding wall of said shaft and the inner side of the surrounding wall of said port.

14. The valve according to claim 11, said valve being configured to enable, when said shaft being at said second position and when said auxiliary opening being covered:
water flow, flowing into said port via a second conduit of said port, leaving said port via a fourth conduit through fluid passage between the outer side of the surrounding wall of said shaft and the inner side of the surrounding wall of said port.

15. The valve according to claim 1, wherein said valve is an air water channel valve.
